# EUROPEAN PATENT APPLICATION

(11) **EP 2 428 209 A1**
(43) Date of publication of application: **14.03.2012**
(21) Application number: 09844384.9
(22) Date of filing: 17.08.2009
(51) Int. Cl.: A61K 31/27

(54) **DRUG SUITABILITY ASSESSMENT METHOD FOR PREVENTION OR TREATMENT OF ANXIETY DISORDERS USING CHOLINERGIC TYPE II THETA RHYTHM**

(30) Priority: 06.05.2009 KR 20090039140
(71) Applicant: Korea Institute of Science and Technology, Seongbuk-gu, Seoul 136-792 (KR)
(72) Inventor: SHIN, Hee-Sup, Uiwang-si Gyeonggi-do 437-812 (KR); SHIN, Jonghan, Seoul 120-857 (KR); GANGADHARAN, Gireesh, Seoul 136-792 (KR); KIM, Seong-Wook, Yongin-si Gyeonggi-do 446-940 (KR); KIM, Duk-Soo, Cheonan-si Chungcheongnam-do 330-110 (KR); LEE, Sukyung, Seoul 135-786 (KR); KIM, Yeon-Soo, Seoul 137-780 (KR)
(74) Representative: advotec.
(86) International application number: PCT/KR2009/004583
(87) International publication number: WO 2010/128717

(57) **Abstract**

The present invention relates to a drug suitability assessment method for the prevention or treatment of anxiety disorders using the cholinergic type II theta rhythm, and, more specifically, to a method for detecting individuals suffering from anxiety disorders induced by an abnormality occurring in the cholinergic system using the type II theta rhythm profile which is based on findings that the cholinergic type II theta rhythm is lower in an animal anxiety model than in normal subjects and that cholinergic drug treatment induces the cholinergic type II theta rhythm to return to normal and reduces anxiety and thereby making it possible to determine if a subject can be appropriately administered with a cholinergic drug and to monitor progress after cholinergic drug treatment.

<110>    Korea Institute of Science and Technology

<120>    Method of Evaluating Suitability for Drug Therapy for the
         prevention and treatment of anxiety disorder using cholinergic
         type II theta rhythm

<130>    9fpo-07-16

<150>    KR10-2009-0039140
<151>    2009-05-06

<160>    2

<170>    KopatentIn 1.71

<210>    1
<211>    21
<212>    DNA
<213>    Artificial Sequence

<220>
<223>    shPLC-beta4-1


<400>    1
gcctcttcaa agtagatgaa t                                                21


<210>    2
<211>    21
<212>    DNA
<213>    Artificial Sequence

<220>
<223>    shPLC-beta4-3


<400>    2
ccgtctccta atgacctcaa a                                                21

## Description

### TECHNICAL FIELD

The present invention relates to a method of evaluating suitability for drug therapy for the prevention or treatment of anxiety disorders using cholinergic type II theta rhythms.

### BACKGROUND ART

Anxiety is both a normal emotion and a psychiatric disorder. Anxiety disorders lead to profound suffering and disability that can markedly disrupt family life as well as the life of the sufferer, especially when associated with avoidance behavior and agoraphobia (Nutt, D.J., CNS Spectr. 10, 49-56, 2005).

Currently, drugs for enhancing a serotoninergic efficacy are used as a drug for the treatment of anxiety disorders (Nutt, D.J., CNS Spectr. 10, 49-56, 2005). Surprisingly, a recent study reported that a drug for increasing acetylcholine neurotransmission in hippocampus attenuates anxiety in some patients (Cummings, J.L. et al., Am. J. Geriatr. Psychiatry. 6, S64-78, 1998; Levy, M.L., et al., Gerontology. 45, S15-22, 1999; Kennedy, D.O. et al., Neuropsychopharmacology. 31, 845-852, 2006). However, a mechanism for this effect has not been disclosed.

It is known that animals have two types of theta rhythm of hippocampus: Type I is a non-cholinergic, serotonine-related theta rhythm; and Type II is a cholinergic theta rhythm (Bland, B.H., Prog. Neurobiol. 26, 1-54, 1986; Shin, J. et al., Proc. Natl. Acad. Sci. U S A. 102, 18165-18170, 2005). Interestingly, attenuated theta rhythms have been observed in human subjects with increased anxiety (Mizuki, Y. et al., Jpn. J. Psychiatry Neurol. 43, 619-626, 1989; Suetsugi, M. et al., Neuropsychobiology. 41, 108-112, 2000). However, there are no studies that establish, regarding anxiety disorders, a physiological meaning of attenuated theta rhythm and that define whether the attenuated theta rhythm is type I or type II.

Anomalies of septal nuclei within the basal forebrain are involved in abnormal information processing at cortical circuits and are responsible for consequent brain dysfunctions, as shown in Alzheimer's disease (Kesner, R.P., et al., Brain Cogn. 9, 289-300, 1989; Colom, L.V., J. Neurochem. 96, 609-623, 2006; Moon, W.J., et al., AJNR Am. J. Neuroradiol. 29, 1308-1313, 2008), Lewy body disease (Fujishiro, H. et al., Acta Neuropathol. 111, 109-114, 2006), frontotemporal dementia (Moon, W.J., et al., AJNR Am. J. Neuroradiol. 29, 1308-1313, 2008), and Parkinson's disease dementia (Dodel, R. et al., J. Neurol. 255, S39-S47, 2008). Medial septum including medial septal nuclei and vertical limb of diagonal band of Broca protrudes toward hippocampus via fornix/fimbria pathway to cause theta oscillation of hippocampus (Bland, B.H., Prog. Neurobiol. 26, 1-54, 1986; Manseau F, et al., J. Neurosci. 28, 4096-4107, 2008). It is considered that theta rhythm shown in human electroencephalogram is generated from corticolimbic interaction that is controlled by hippocampal theta rhythm (Miller, R., Springer-Verlag, Berlin, 1991; Basar, E., et al., Int. J. Psychophysiol. 39, 197-212, 2001; Kahana, M.J., et al., Curr. Opin. Neurobiol. 11, 739-744, 2001; Cantero, J.L., J. Neurosci. 23, 10897-10903, 2003; Gordon, J.A., et al., J. Neurosci. 25, 6509-6519, 2005; Tejada, S. et al., Eur. J. Neurosci. 26,199-206, 2007).

Phospholipase C (PLC)-β is differentiated from PLC-γ and PLC-δ based on structure and activation mechanism. PLC-β acts through G protein-dependent pathways, and the pathway is engaged by the activation of specific isoforms of neurotransmitter receptors that have seven transmembrane-spanning group I metabotropic glutamate receptor (mGluR1 and mGluR5), a serotonergic receptor (5-HT2)(Abe, T. et al., J. Biol. Chem. 267, 13361-13368, 1992), and a muscarinic acetylcholine receptor (M1, M3 and M5)(Gutkind, J.S., et al., Proc. Natl. Acad. Sci. USA 88, 4703-4707 , 1991). Four PLC-β isoforms represented by PLC-β1, PLC-β2, PLC-β3 and PLC-β4 each have a unique distribution pattern in the brain (Kim, D. et al., Nature 389, 290-293, 1997; Watanabe, M. et al., Eur. J. Neurosci. 10, 2016-2025, 1998). PLC-β4 is expressed in the soma and dendrites of neurons in the medial septum, one of the three brain regions (hippocampus, amygdala, and septum) (Treit, D. & Menard, J., Behav. Neurosci.111, 653-658, 1997; Gray, J.A. & McNaughton, N. The neuropsychology of anxiety, Ed 2. New York: Oxford UP, 2000) implicated in anxiety behaviors (Watanabe, M. et al., Eur. J. Neurosci. 10, 2016-2025, 1998; Nakamura, M. et al., Eur. J. Neurosci. 20, 2929-2944, 2004). The medial septum is also a nodal point involved in generating hippocampal theta rhythms (Bland, B.H., Prog. Neurobiol. 26, 1-54, 1986). These observations present a possibility that PLC-β4 may be critically involved in linking anxiety behaviors and theta rhythm heterogeneity.

However, up to now, the relationship among cholinergic drugs, theta rhythm, and anxiety behaviors has not been revealed.

So, the inventors of the present application studied the relationship between anxiety behaviors and theta rhythm by using PLC-β4-knock-out (PLC-β4^{-*l*-}) mouse, and confirmed that a global deletion or a medial septum-selective knock-down of PLC-β4 attenuated cholinergic type II theta rhythm and increased anxiety behaviors. Also, it was confirmed that when the PLC-β4-knock-out mouse was treated with a cholinergic enhancer, cholinergic type II theta rhythm anomalies and anxiety behaviors all were cured. These results show that measuring cholinergic type II theta rhythm may provide an effective guide line for the treatment of anxiety disorders.

### DETAILED DESCRIPTION OF THE INVENTION

### TECHNICAL PROBLEM

The present invention provides a method of screening an effective therapeutic agent for the treatment of an anxiety disorder subject and a method of monitoring a progress of the anxiety disorder after a cholinergic drug treatment, by using a cholinergic type II theta rhythm profile,.

### TECHNICAL SOLUTION

According to an aspect of the present invention, there is provided a method of evaluating suitability for drug therapy for the prevention or treatment of anxiety disorders by using cholinergic type II theta rhythms.

According to another aspect of the present invention, there is provided a method of monitoring prognosis of anxiety disorders after the administration of a cholinergic enhancer by using cholinergic type II theta rhythms.

According to another aspect of the present invention, there is provided a method of diagnosing anxiety disorders by using cholinergic type II theta rhythms.

According to another aspect of the present invention, there is provided a method of screening an agent that prevents or treats anxiety disorders by using cholinergic type II theta rhythms.

According to another aspect of the present invention, there is provided a kit for evaluating suitability for drug therapy for the prevention or treatment of anxiety disorder, wherein the kit includes an electroencephalogram recorder for analyzing cholinergic type II theta rhythms.

According to another aspect of the present invention, there is provided a kit for monitoring prognosis of anxiety disorders after administration of a cholinergic enhancer, wherein the kit includes an electroencephalogram recorder for analyzing cholinergic type II theta rhythms.

Hereinafter, the terms used herein will be defined below.

The term "cholinergic enhancer" used herein refers to a composition for enhancing or regulating cholinergic neurotransmission by allosteric sensitization, the direct activation of a cholinergic receptor, the activation of intracellular pathways between related cells through second messenger cascades, or inhibition of cholinesterases. The inhibition of cholinesterases may increase the synapse concentration of acetylcholine (ACh) to enhance and extend the activation of acetylcholine in a muscarinic acetylcholine receptor (mAChR) and a nicotinic acetylcholine receptor (nAChR).

The term "anxiety" used herein refers to a fear of an undefined subject, that is, an offensive and agonizing emotional reaction we have in a threatening and dangerous situation that may result in negative results.

The term "anxiety disorders" used herein refers to a mental disorder in which anxiety arises without any reason and a level of anxiety is too high. That is, this term refers to a case in which excessive psychological agony arises due to morbid anxiety or serious difficulty occurs in adapting the real life.

The term "prevention" used herein refers to any behavior that inhibits symptoms of anxiety disorders or delaying progress of anxiety disorders due to the administration of a composition according to the present invention.

The term "treatment" used herein refers to any behavior that attenuates symptoms of anxiety disorders or changes the symptoms in a beneficial way due to the administration of a composition according to the present invention.

The term "administration" used herein refers to supplying a composition according to the present invention to a subject by using an appropriate method.

The term "subject" used herein refers to an animal, such as humans, monkeys, dogs, goats, pigs, or mice, which has a disease of which symptoms of anxiety disorders can be attenuated by the administration of a composition according to the present invention.

The term "effective amount" used herein refers to an amount that is sufficient for the treatment of a disease at a rational benefit or risk ratio that is applicable for medication treatment. The effective amount may be determined according to a disease of a subject, a level of severeness, the activation of drug, sensitivity to drug, an administration time, an administration pathway, a discharge ratio, a treatment duration, a drug that is simultaneously used, and other factors known the medication field.

Hereinafter, the present invention will be described in detail.

The present invention provides a method of evaluating suitability for drug therapy for the prevention or treatment of anxiety disorders by using cholinergic type II theta rhythms.

In detail, the method may include the following steps, but is not limited thereto:
1) recording a cholinergic type II theta rhythm from a subject having an anxiety disorder;
2) selecting a subject of which an amplitude of cholinergic type II theta rhythm is less than that of a normal subject; and
3) evaluating the subject as a subject that needs an cholinergic enhancer as an agent for the treatment of the anxiety disorder.

Regarding the method, the anxiety disorder of step 1) is selected from the group consisting of a panic disorder, phobia, an obsessive-compulsive disorder, a posttraumatic stress disorder, an acute stress disorder, a generalized anxiety disorder, and a separation anxiety disorder, but is not limited thereto.

The panic disorder is an extreme anxiety symptom in which a fear arises suddenly without any reason, followed by suffocation or a strong heart beat. That is, the panic disorder is a disorder that has several events of panic attack and that may occur due to a fatigue, excitement, sexual behaviors, or emotional impacts. However, typically, the panic disorder cannot be predicted and suddenly occurs.

The phobia refers to an anxiety disorder characterized by avoiding a particular situation or subject due to severe anxiety and fear of the particular situation or subject. 1) Specific phobia is a disorder characterized by recurring an irrational fear of and avoidance behaviors for a particular subject or situation. 2) Social Phobia is a disorder characterized by repeatedly showing avoidance behaviors due to a fear of a social situation where interaction occurs among people. 3) Agoraphobia is a disorder characterized by repeatedly showing a fear of a particular place or situation.

The obsessive-compulsive disorder is an anxiety disorder characterized by recurring an undesired thinking, that is, obsessions, and behaviors.

The posttraumatic stress disorder is an anxiety disorder characterized by a persistent anxiety in response to a terrifying event.

The acute stress disorder is an anxiety disorder that shows symptoms similar to those of the posttraumatic stress disorder and that is characterized by showing dissociative symptoms in response to a traumatic event.

The generalized anxiety disorder is an anxiety disorder characterized by chronic anxiety and excessive worry with respect to various situations.

The separation anxiety disorder is a condition in which an individual experiences excessive anxiety regarding separation from people to whom the individual has a strong emotional attachment. If the separation anxiety excesses a normal range and interferes with a normal social life, this can be said as a morbid state. In this respect, the separation anxiety disorder refers to a case in which the separation anxiety is excessive and thus interferes with normal activities.

Regarding the method, the cholinergic type II theta rhythm of step 1) may be measured by electroencephalogram (EEG). However, the measuring method is not limited thereto.

The cholinergic type II theta rhythm may occur during urethane anesthesia, alert immobility, or passive whole-body rotation (Bland, B.H., Prog. Neurobiol. 26, 1-54, 1986; Shin, J. et al., Proc. Natl. Acad. Sci. U S A. 102, 18165-18170, 2005), and the type I theta rhythm may occur during mobile activities, for example, working or running (Bland, B.H., Prog. Neurobiol. 26, 1-54, 1986; Shin, J. & Talnov, A., Brain Res. 897, 217-221, 2001).

Regarding the method, the cholinergic enhancer of step 3) may be a composition that increases an amount of acetylcholine neurotransmitter in hippocampus and medial septum in the brain. For example, the cholinergic enhancer may be an acetylcholinesterase inhibitor, a drug that enhances acetylcholine neurotransmission by inhibiting acetylcholinesterase secreted from nerve endings, but is not limited thereto. That is, other than the acetylcholinesterase inhibitor, a cholinergic enhancer component that enhances or regulates allosteric sensitization, the direct activation of a cholinergic receptor, or the activation of intracellular pathways between related cells through second messenger cascades.

The acetylcholinesterase inhibitor may be any one selected from the group consisting of rivastigmine, donepezil, galantamine, tacrine, metrifonate, physostigmine, neostigmine, pyridostigmine, ambenonium, demarcarium, edrophonium, huperzine A, and onchidal, but is not limited thereto.

For example, the acetylcholinesterase inhibitor may be any one selected from the group consisting of rivastigmine, donepezil, galantamine, and tacrine. For example, the acetylcholinesterase inhibitor is rivastigmine, but is not limited thereto (Van Dam, D., et al., Psychopharmacology 180, 177-190, 2005; Cerbai, F. et al., Eur. J. Pharmacol. 572, 142-150, 2007; Kosasa, T., et al., Eur. J. Pharmacol. 380, 101-107, 1999; Scali, C. et al., J. Neural. Transm. 109, 1067-1080, 2002; Liang, Y.Q. et al., Acta. Pharmacol. Sin. 27, 1127-1136, 2006; Enz, A. et al., Prog. Brain Res. 98, 431-438, 1993; Weinstock, M. et al., J. Neural Transm. Suppl. 43, 219-225, 1994).

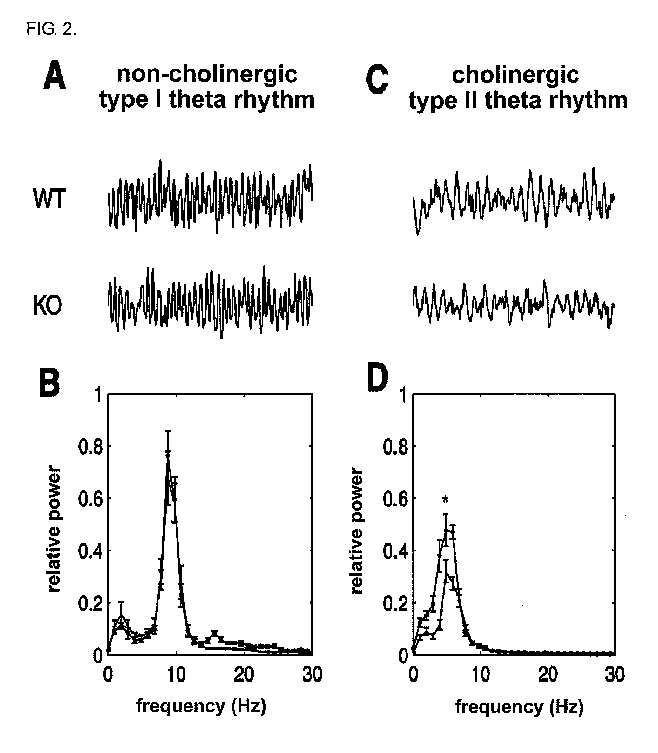

To confirm the relationship between theta rhythm and anxiety behaviors, hippocampal EEG was performed on as an anxiety behavior animal model a PLC-β4 knock-out (PLC-β4 ^{-*l*-}) mouse (see KR10-2008-0007202) and a wild-type mouse to analyze and compare their theta rhythm profiles. As a result, regarding the non-cholinergic type I theta rhythm, there is no significant difference between the PLC-β4 ^{-/-} mouse and the wild-type mouse. However, regarding the cholinergic type II theta rhythm, the theta amplitude of the PLC-β4 ^{-/-} mouse was significantly decreased compared to the theta amplitude of the wild-type mouse (see FIG. 2). Accordingly, it was confirmed that the cholinergic type II theta rhythm is related to the induction of anxiety.

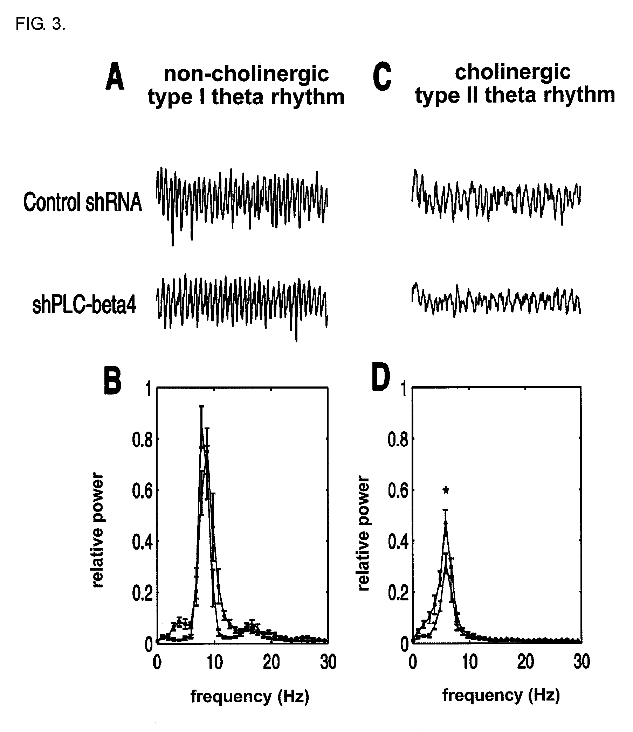

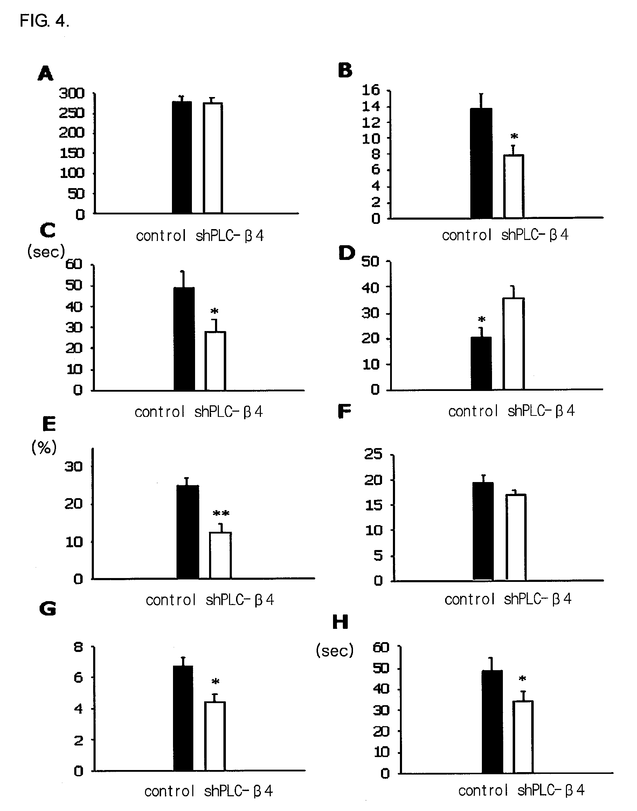

Also, to confirm the relationship between PLC-β4 and the cholinergic type II theta rhythm in the medial septum, lentiviral vectors expressing PLC-β4-targeting shRNA (shPLC-β4) and control shRNA were injected into the medial septum, and then anxiety behaviors thereof were evaluated and EEG was recorded. As a result, the wild-type mouse infected with lentivirus expressing shPLC-β4 showed a significant decrease in the amplitude of cholinergic type II theta rhythm, compared to the wild-type mouse infected with control shRNA (see FIG. 3), and also showed significantly high anxiety behaviors (see FIG. 4). Accordingly, it can be confirmed that PLC-β4 of the medial septum regulates cholinergic type II theta rhythm and the decrease in the cholinergic type II theta rhythm amplitude induces anxiety behaviors.

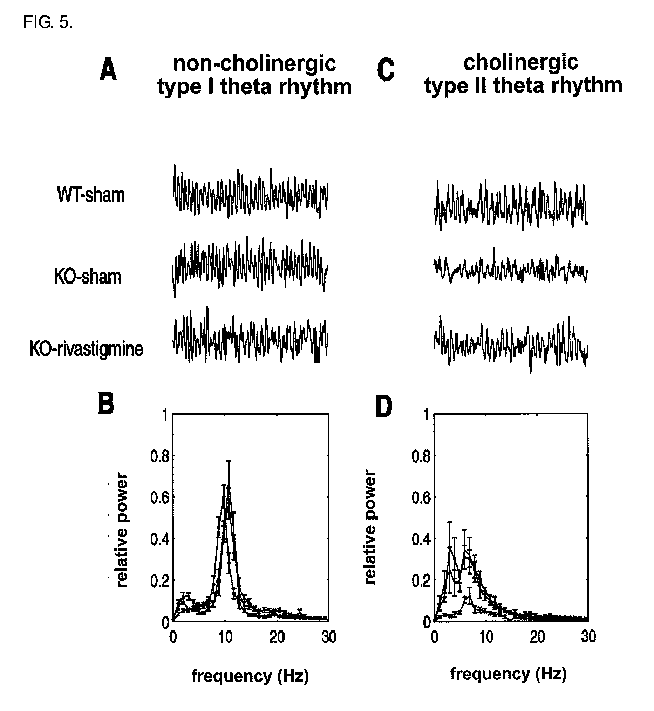

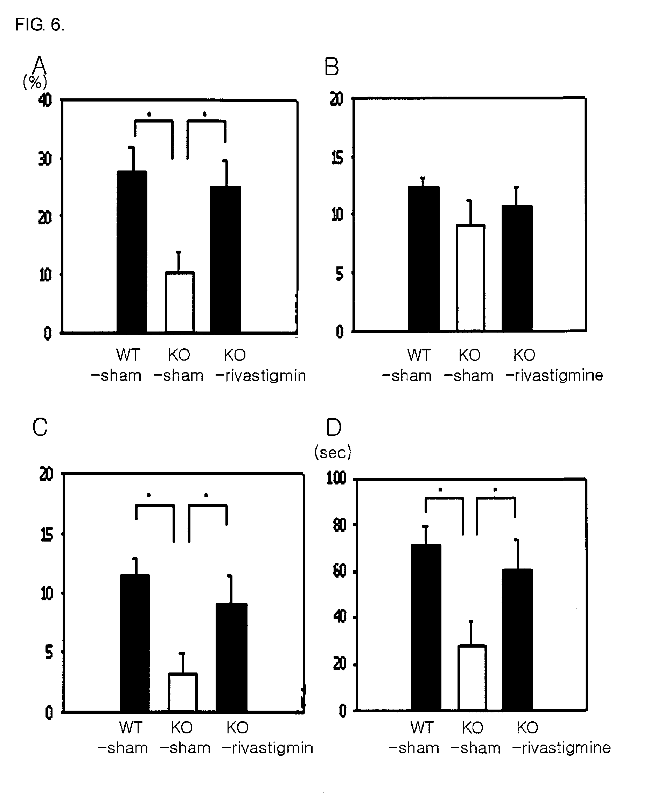

Also, to confirm whether anxiety behaviors are normalized by increasing the cholinergic type II theta rhythm amplitude by using a cholinergic enhancer, rivastigmine and saline were respectively administered to PLC-β4 ^{-/-} mouse and wild-type mouse and then anxiety behaviors thereof were evaluated and EEG was recorded. As a result, the rivastigmine administration restored to levels of normal cholinergic type II theta rhythm and normal anxiety behaviors in PLC-β4 ^{-/-} mouse (see FIGS. 5 and 6). Accordingly, it can be confirmed that the cholinergic enhancer restores the cholinergic type II theta rhythm and normalize anxiety behaviors.

As a drug for the effective treatment of anxiety disorders, a serotonergic drug, a GABA drug, and a cholinergic drug are known. However, due to a variety of anxiety behavioral subject, biological markers that enable prescription of a right drug from among them to a right subject have not been developed. Accordingly, according to the law of 'trial and error', all of the drugs are once used and then from the result, the most effective drug is selected.

However, according to the present invention, it is determined by measuring characteristics of cholinergic type II theta rhythm whether anxiety behavioral subject needs a cholinergic drug. Accordingly, the present invention enables the evaluation of drug suitability for a subject.

Also, the present invention provides a method of monitoring prognosis of anxiety disorders after the administration of a cholinergic enhancer, by using cholinergic type II theta rhythms.

In detail, the method includes the following steps, but is not limited thereto:
1) administrating an effective amount of a cholinergic enhancer to a subject having an anxiety disorder;
2) recording a cholinergic type II theta rhythm from the subject; and
3) evaluating a restoration level of the amplitude of the cholinergic type II theta rhythm compared with that of a normal subject as a recovery level of the anxiety disorder.

Regarding the method, the cholinergic enhancer of step 3) may be a composition that increases an amount of acetylcholine neurotransmitter in hippocampus and medial septum in the brain. For example, the cholinergic enhancer may be an acetylcholinesterase inhibitor, a drug that enhances acetylcholine neurotransmission by inhibiting acetylcholinesterase secreted from nerve endings, but is not limited thereto. That is, other than the acetylcholinesterase inhibitor, a cholinergic enhancer component that enhances or regulates allosteric sensitization, the direct activation of a cholinergic receptor, or the activation of intracellular pathways between related cells through second messenger cascades.

The acetylcholinesterase inhibitor may be any one selected from the group consisting of rivastigmine, donepezil, galantamine, tacrine, metrifonate, physostigmine, neostigmine, pyridostigmine, ambenonium, demarcarium, edrophonium, huperzine A, and onchidal, but is not limited thereto.

Regarding the method, the anxiety disorder of step 1) is selected from the group consisting of a panic disorder, phobia, an obsessive-compulsive disorder, a posttraumatic stress disorder, an acute stress disorder, a generalized anxiety disorder, and a separation anxiety disorder, but is not limited thereto.

Regarding the method, the cholinergic type II theta rhythm of step 2) may be measured by analyzing hippocampal EEG during urethane anesthesia, alert immobility, or passive whole-body rotation. However, the measurement method is not limited thereto.

According to the present invention, an anxiety behavioral subject has attenuated cholinergic type II theta rhythm compared to a normal subject, and when the cholinergic drug is administered, the cholinergic type II theta rhythm is restored to a normal level, thereby recovering anxiety behaviors. Accordingly, the relationship among the cholinergic type II theta rhythm, the cholinergic drug, and anxiety behaviors is confirmed. Based on the confirmation, a progress of anxiety disorders after the administration of cholinergic drug to anxiety behavioral subject is monitored by evaluating characteristics of the cholinergic type II theta rhythms.

Also, the present invention provides a method of diagnosing anxiety disorders by using the cholinergic type II theta rhythms.

In detail, the method includes the following steps, but is not limited thereto:
1) recording a cholinergic type II theta rhythm from a subject; and
2) determining a subject that has a lower amplitude of a cholinergic type II theta rhythm than that of a normal subject as a subject that is prone to develop an anxiety disorder.

Regarding the method, the cholinergic type II theta rhythm of step 1) may be measured by analyzing hippocampal EEG during urethane anesthesia, alert immobility, or passive whole-body rotation. However, the measurement method is not limited thereto.

According to the present invention, an anxiety behavioral subject has attenuated cholinergic type II theta rhythm compared to a normal subject, and when the cholinergic drug is administered, the cholinergic type II theta rhythm is restored to a normal level, thereby recovering anxiety behaviors. Accordingly, the relationship among the cholinergic type II theta rhythm, the cholinergic drug, and anxiety behaviors is confirmed. Based on the confirmation, by measuring characteristics of the cholinergic type II theta rhythm, a subject that has a lower amplitude of the cholinergic type II theta rhythm than that of a normal subject is evaluated as an anxiety behavioral subject.

Also, the present invention provides a method of screening a drug for the prevention or treatment of anxiety disorders by using the cholinergic type II theta rhythms.

In detail, the method includes the following steps, but is not limited thereto:
1) administering a test material to a subject having an anxiety disorder;
2) measuring a cholinergic type II theta rhythm of the subject; and
3) selecting a material that attenuates the anxiety disorder by comparing an amplitude of the cholinergic type II theta rhythm of the subject with that of a normal subject.

Regarding the method, the anxiety disorder of step 1) is selected from the group consisting of a panic disorder, phobia, an obsessive-compulsive disorder, a posttraumatic stress disorder, an acute stress disorder, a generalized anxiety disorder, and a separation anxiety disorder, but is not limited thereto.

Regarding the method, the cholinergic type II theta rhythm of step 2) may be measured by analyzing hippocampal EEG during urethane anesthesia, alert immobility, or passive whole-body rotation. However, the measurement method is not limited thereto.

According to the present invention, an anxiety behavioral subject has attenuated cholinergic type II theta rhythm compared to a normal subject, and when the cholinergic drug is administered, the cholinergic type II theta rhythm is restored to a normal level, thereby recovering anxiety behaviors. Accordingly, the relationship among the cholinergic type II theta rhythm, the cholinergic drug, and anxiety behaviors is confirmed. Based on the confirmation, by measuring a cholinergic type II theta rhythm from an anxiety disorder subject treated with a drug, a drug that effectively restores the cholinergic type II theta rhythm to a normal level can be screened.

Also, the present invention provides a kit for evaluating suitability for drug therapy for the prevention or treatment of anxiety disorder, wherein the kit includes an EEG recorder for analyzing the cholinergic type II theta rhythms.

Also, the present invention provides a kit for monitoring prognosis of anxiety disorders after the administration of a cholinergic enhancer, wherein the kit includes an EEG recorder for analyzing the cholinergic type II theta rhythms.

Also, the present invention provides a kit for diagnosing anxiety disorders, wherein the kit includes an EEG recorder for analyzing the cholinergic type II theta rhythms.

The present invention a kit for screening an agent for the prevention or treatment of anxiety disorders, wherein the kit includes an EEG recorder for analyzing the cholinergic type II theta rhythms.

According to the present invention, an anxiety behavioral subject has attenuated cholinergic type II theta rhythm compared to a normal subject, and when the cholinergic drug is administered, the cholinergic type II theta rhythm is restored to a normal level, thereby recovering anxiety behaviors. Accordingly, the relationship among the cholinergic type II theta rhythm, the cholinergic drug, and anxiety behaviors is confirmed. Based on the confirmation, it can be confirmed that analyzing the cholinergic type II theta rhythm by using an EEG recorder is useful for evaluation for suitability for drug therapy for the prevention or treatment of anxiety disorders, monitoring prognosis of anxiety disorders after the administration of the cholinergic enhancer, diagnosis of anxiety disorders, and screening an agent for the prevention or treatment of anxiety disorders.

### ADVANTAGEOUS EFFECTS

The present invention may be useful,in studying a mechanism between a cholinergic drug, type II theta rhythm, and anxiety behaviors based on the confirmation that cholinergic type II theta rhythm is related to anxiety behaviors. Also, as a drug for the effective treatment of anxiety disorders, a serotonergic drug, a GABA drug, and a cholinergic drug are known, but due to a variety of anxiety behavioral subjects, biological markers that enable prescription of a right drug from among them to a right subject have not been developed. Accordingly, according to the law of 'trial and error', all of the drugs are once used and then from the result, the most effective drug is selected. However, according to the present invention, it can be determined by using characteristics of the cholinergic type II theta rhythm that use of a cholinergic drug is suitable for what kind of anxiety behavior subject. Also, the progress of anxiety disorders after the administration of a cholinergic drug may be monitored by using characteristics of the cholinergic type II theta rhythms.

### DESCRIPTION OF THE DRAWINGS

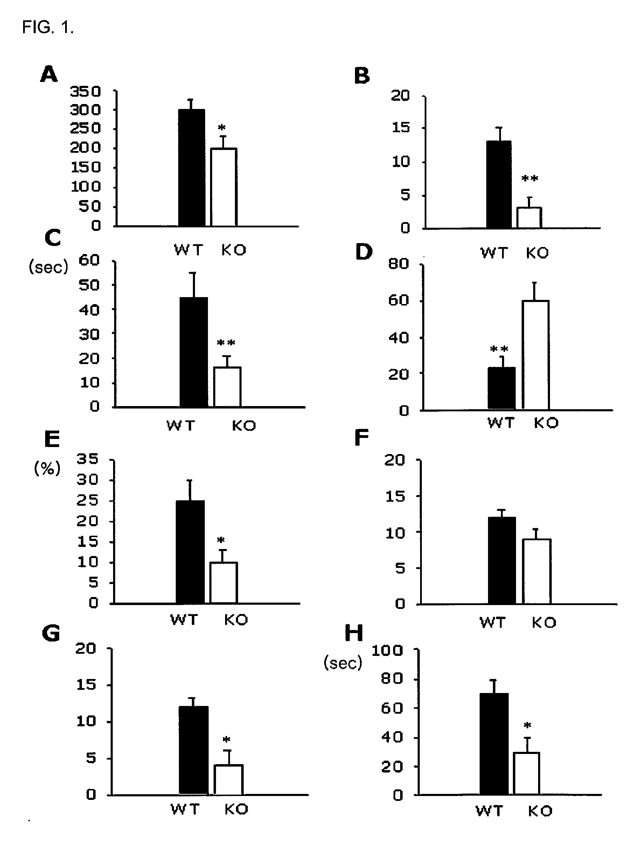

FIG. 1 shows results of open field, elevated plus-maze, and light-dark transition anxiety behavior tests which were performed on PLC-β4 ^{-/-} mice to confirm occurrence of anxiety behaviors:
   (A) Locomotor activity in an open field;
   (B) Number of central cross in an open field;
   (C) Time in the central sector in an open field;
   (D) Thigmotaxis;
   (E) Percent entries into the open arms in elevated plus-maze;
   (F) Total number of entries in elevated plus-maze;
   (G) Light/dark transition number; and
   (H) Total time in the light compartment.
FIG. 2 illustrates a non-cholinergic type I theta rhythm and a cholinergic type II theta rhythm which are obtained by analyzing EEG of wild-type mice (WT) and PLC-β4 ^{-/-} mice (KO), wherein this experiment was performed to identify a theta rhythm state of PLC-β4 ^{-/-} mice:
   (A) EEG waveforms during walking for wild-type mice and PLC-β4 ^{-/-} mice ;
   (B) Averaged power spectra of EEG waveforms during walking for wild-type mice and PLC-β4 ^{-/-} mice;
   (C) EEG waveforms during urethane anesthesia for wild-type mice and PLC-β4 ^{-/-} mice; and
   (D) Averaged power spectra of EEG waveforms during urethane
      anesthesia for wild-type mice and PLC-β4 ^{-/-} mice.
FIG. 3 shows EEG assay results of wild-type mice injected with lentiviruses expressing shPLC-β4 and control shRNA, wherein this experiment was performed to confirm that medial septum-selective PLC-β4 knockdown replicates the theta rhythm heterogeneity phenotype of PLC-β4 ^{-/-} mice:
   (A) EEG waveforms during walking for control shRNA mice and shPLC-β4 mice;
   (B) Averaged amplitude spectra of the EEG waveforms recorded during walking for control shRNA mice and shPLC-β4 mice;
   (C) EEG waveforms during urethane anesthesia for control shRNA mice and shPLC-β4 mice; and
   (D) Averaged amplitude spectra of the EEG waveforms recorded during urethane anesthesia for control shRNA mice and shPLC-β4 mice.
FIG. 4 shows results of open field, elevated plus-maze, and light-dark transition anxiety behavior tests which were performed on wild-type mice injected with lentiviruses expressing shPLC-β4 and control shRNA, wherein this experiment was performed to confirm that medial septum PLC-β4 knockdown causes anxiety behaviors of PLC-β4 ^{-/-} mice:
   (A) Locomotor activity in an open field;
   (B) Number of central cross in an open field;
   (C) Time in the central sector in an open field;
   (D) Thigmotaxis;
   (E) Percent entries into the open arms in elevated plus-maze;
   (F) Total number of entries in elevated plus-maze;
   (G) Light/dark transition number; and
   (H) Total time in the light compartment.
FIG. 5 shows EEG assay results of wild-type mice and PLC-β4 ^{-/-} mice with intraperitoneal injection with rivastigmine or saline (WT-sham: wild-type mice injected with saline; KO-sham: PLC-β4 ^{-/-} mice injected with saline; and KO-rivastigmine: PLC-β4 ^{-/-} mice injected with rivastigmine), wherein this experiment was performed to confirm that rivastigmine normalizes the theta rhythm heterogeneity phenotype of PLC-β4 ^{-/-} mice:
   (A) EEG waveforms during walking for WT-sham, KO-sham and KO-rivastigmine;
   (B) Averaged power spectra of EEG waveforms recorded during walking for WT-sham, KO-sham, and KO-rivastigmine;
   (C) EEG waveforms during alert-immobility for WT-sham, KO-sham, and KO-rivastigmine;
   (D) Averaged power spectra of EEG waveforms recorded during alert-immobility for WT-sham, KO-sham, and KO-rivastigmine.
FIG. 6 shows results of open field, elevated plus-maze, and light-dark transition anxiety behavior tests which were performed on wild-type mice and PLC-β4 ^{-/-} mice intraperitoneally injected with rivastigmine or saline, wherein this experiment was performed to confirm that tivastigmine restores increased anxiety behavior of PLC-β4 ^{-/-} mice (WT-sham: wild-type mice; KO-sham injected with saline: PLC-β4 ^{-/-} mice injected with saline; and KO-rivastigmine: PLC-β4 ^{-/-} mice injected with rivastigmine):
   (A) Percent entries into the open arms in elevated plus-maze;
   (B) Total number of entries in elevated plus-maze;
   (C) Light/dark transition number; and
   (D) Total time in the light compartment.

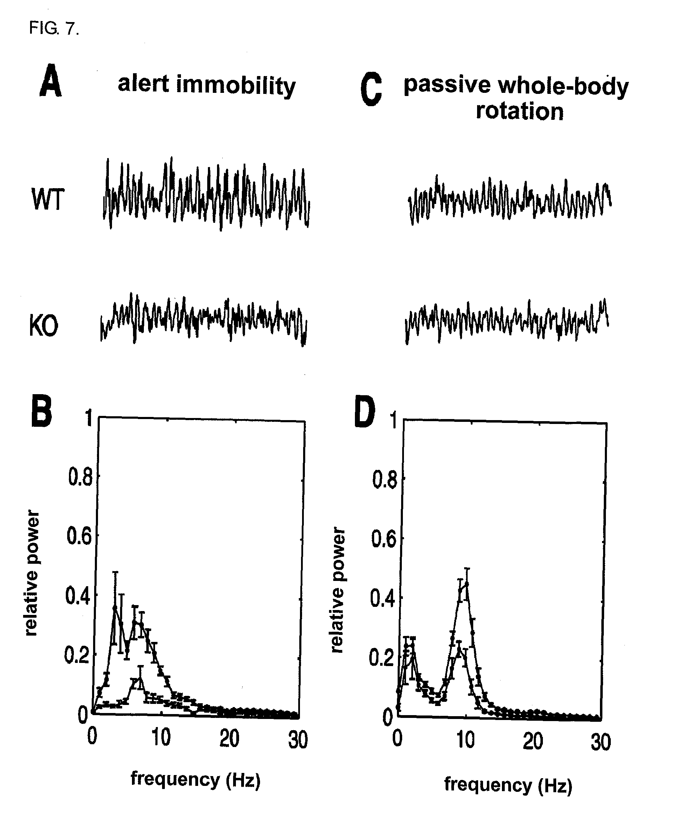

FIG. 7 illustrates cholinergic type II theta rhythms obtained by analyzing EEG during alert immobility and passive whole-body rotation for wild-type mice (WT) and PLC-β4 ^{-/-} mice (KO), wherein this experiment was performed to identify cholinergic type II theta rhythm states during alert immobility and passive whole-body rotation:
   (A) EEG waveforms during alert immobility for wild-type mice (WT) and PLC-β4 ^{-/-} mice (KO);
   (B) Averaged power spectra of EEG waveforms during alert immobility for wild-type mice (WT) and PLC-β4 ^{-/-} mice (KO);
   (C) EEG waveforms during passive whole-body rotation for wild-type mice (WT) and PLC-β4 ^{-/-} mice (KO); and
   (D) Averaged power spectra of EEG waveforms during passive whole-body rotation for wild-type mice (WT) and PLC-β4 ^{-/-} mice (KO).

### BEST MODE

Hereinafter, the present invention will be described in detail with reference to examples.

However, the following examples are presented for illustrative purpose only and the present invention is not limited thereto.

### <Example 1> Anxiety behavior assay on PLC-β4 ^{-/-} mice

The inventors of the present invention performed open-field thigmotaxis, elevated plus-maze, and light/dark transition between 9:00 A.M. and 5:00 P.M. using adult (8- to 16-week-old) mice to confirm that PLC-β4 ^{-/-} mice show increased anxiety behaviors.

For statistic analysis, differences between groups were compared using Student's *t* test after confirming that data sets were normally distributed. Behavioral data for PLC-β4^{-/-} mice and wild-type mice were analyzed by ANOVA followed by Tukey's post-hoc test to determine differences among groups.

### <1-1> Preparation of PLC-β4^{-/-} mice

PLC-β4^{-/-} mice was prepared in the same manner as described in KR 10-2008-0007202. In detail, PLCβ4^{+/+} and PLCβ4-/- mice was obtained by crossing C57BL/6J(N8)PLC-β4^{+/-} and 129S4/SvJae(N8)PLC-β4^{+/-} mice in step F1. The geno types were determined using PCR analyses as described previously (Kim, D. et al., Nature 389, 290-293, 1997). Animal care and handling procedures followed institutional guidelines (KIST, Korea). Mice were maintained with *ad libitum* access to food and water under a 12 h light/dark cycle, and under a specific pathogen free (SPF) environment in which the temperature and humidity were maintained at 22°C and 55%, respectively.

### <1-2> Anxiety Behavior Test

Open field thigmotaxis was assessed by placing mice in the center of an open field apparatus (40 x 40 x 40 cm) under dim lighting. During a 10 min observation period, locomotor activity, number of central crosses, and time spent in the central sector of the open field were recorded and analyzed by PC-based video behavior assay system (TSE, Bad Homburg, Germany). Thigmotaxis index was defined as a ratio of the number of entries into the central part of a testing arena to the locomotor activity. The thigmotaxis index was calculated for each mouse separately and used to calculate means and SEMs for a given experimental group (Treit, D. & Fundytus, M., Pharmacol. Biochem. Behav. 31, 959-962, 1988; Sienkiewicz-Jarosz, H. et al., J Neural Transm.107, 1403-1412, 2000).

In the open-field assay, PLC-β4 ^{-/-} mice showed reduced locomotion in the open field compared with wild-type mice (FIG. 1A; *P* < 0.05, Student's *t-*test). The PLC-β4 ^{-/-} mice crossed the center of the open field less frequently than did wild-type mice (FIG. 1B; *P* < 0.05, Student's *t*-test), and spent less time in the central sector of the open field (FIG. 1C; *P* < 0.05, Student's *t-*test). Accordingly, it was confirmed that thigmotaxis, a mouse's natural tendency to stay near the perimeters of a novel environment (Treit, D. & Fundytus, M., Pharmacol. Biochem. Behav. 31, 959-962, 1988) was enhanced in PLC-β4 ^{-/-} mice compared with wild-type mice (FIG. 1D; *P* < 0.001, Student's *t*-test).

The elevated plus-maze consists of two open and two enclosed arms of the same size (45 □ 5 cm) with walls 15 cm high. The arms, constructed of black acrylic, radiate from a central platform (5□5 cm) to form a plus sign. The entire apparatus was elevated to a height of 30 cm above floor level. Each mouse was placed in the central platform facing one of the open arms. The number of entries into the open and closed arms and the time spent on the open and closed arms were recorded during a 5 min test period (Parks, C.L. et al., Proc. Natl. Acad. Sci. USA 95, 10734-10739; 1998; Ramboz, S. et al., Proc. Natl. Acad. Sci. USA 95, 14476-14481, 1998; Gross, C. et al., Nature 416, 396-400, 2002).

In the elevated plus-maze, PLC-β4 ^{-/-} mice made fewer entries into the open arms compared with wild-type mice (FIG. 1E; *P* < 0.001, Student's *t-*test), and the total number of entries did not differ between the two groups (FIG. 1 F; *P* < 0.001, Student's *t*-test).

The apparatus used for the light/dark transition test consisted of a cage (25 x 40 x 20 cm) divided into two compartments by a black partition containing a small opening that allows the mouse to move from one compartment to the other. One compartment, comprising two-thirds of the surface area, was made of white plastic and was brightly illuminated; the adjoining smaller compartment was black and dark. Mice were placed in the dark compartment and allowed to move freely between the two chambers for 5 min. The number of transitions between the two compartments, time spent in each chamber, and latency to the first transition were recorded (Welch, J.M. et al., Nature 448, 894-900, 2007).

In the light/dark box test, PLC-β4 ^{-/-} mice made fewer transitions from the dark to the light compartment than did wild-type mice (FIG. 1 G) ( *p _* 0.01, Student's *t* test). Consistent with this, the time spent in the light chamber was significantly shorter for PLC-β4 ^{-/-} mice than for wild-type mice (FIG. 1H; *P* < 0.001, Student's *t*-test).

### <Example 2> Profile of theta rhythm in PLC-β4 ^{-/-} mice

To determine whether the theta rhythm profile is changed in PLC-β4 ^{-/-} mice and, if so, whether the difference is attributable to non-cholinergic serotonine-related type I or cholinergic type II theta rhythm, the inventors of the present application examined hippocampal EEG in 10- to 14-week male PLC-β4 ^{-/-} mice and wild-type mice using previously established protocols (Bland, B.H., Prog. Neurobiol. 26, 1-54, 1986; Shin, J. et al., Proc. Natl. Acad. Sci. U S A. 102, 18165-18170, 2005).

For statistic analysis, differences between groups were compared using Student's *t* test after confirming that data sets were normally distributed. Behavioral data for PLC-β4^{-/-} mice and wild-type mice were analyzed by ANOVA followed by Tukey's post-hoc test to determine differences among groups.

### <2-1> Local field potential recordings in vivo

For electrode implantation, the animals were anesthetized with pentobarbital (50 ml/kg, i.p.) and held in a stereotaxic apparatus with bregma and lambda in the same horizontal plane. Hippocampal EEG recordings were performed using Teflon-coated tungsten electrodes (150 um) implanted in the hippocampal fissure with grounding over the cerebellum (from bregma, 2.0 mm anteroposterior, 1.2 mm mediolateral, and 1.8 mm dorsoventral). The position of the electrodes was verified by light microscopy in Nissl-stained sections according to published protocols. Field potential was amplified (x 1,000), bandpass-filtered (bandpass-filtered)(0.1-100 Hz), digitized with 12-bit resolution continuously at 1 kHz sampling, and recorded on a personal computer.

### <2-2> Analysis of hippocampal electrical activity data

The EEG EEG data were collected in 4 s segments and fast-Fourier transformed (FFT). The data were continuously monitored for movement artifacts, and the recording of each segment was manually verified by the experimenter, blinded to the genotype of the animals. All segments collected during mouse movements and those in which the amplitude of amplified EEG signals exceed a maximum of ± 1.25V were discarded. To compare the EEG spectral characteristics of hippocampal electrical activities, EEG spectral power in 1 Hz bins was calculated using FFT (Hamming window) of each 4 s epoch. These analyses were performed on recordings from these intervals in 100 trials from each group. Powers in the 0-30 Hz range were averaged in groups across each behavioral state, and the mean values were plotted in 1 Hz bins. The averaging process for power spectrums used a normalization procedure that involved dividing by the combined SD of EEG raw data for the two comparison states (e.g., PLC-β4^{-/-} mice and wild-type mice). The peak power under different conditions was used for comparison purposes because the in vivo theta rhythm has a clear and sharp peak frequency that distinguished it from other in vivo EEG rhythms, such as delta- and gamma-band activities and peak power provides more accurate information than total power in the theta frequency range.

### <2-3> Classification of non-cholinergic type I theta and cholinergic type II theta rhythm

Cholinergic and noncholinergic theta rhythms associated with different behaviors were recorded. Non-cholinergic, serotonine-related type I theta rhythms can be distinguished from cholinergic type II theta rhythms using muscarinic antagonists [e.g., atropine or scopolamine], which can abolish cholinergic type II theta rhythms when injected into the animals, but leave non-cholinergic type I theta rhythms relatively unaffected. In addition, the use of different behaviors to induce theta rhythms can discriminate betweennon-cholinergic, serotonine-related type I theta rhythms and cholinergic type II theta rhythms. For example, cholinergic type II theta rhythms are generated normally during urethane anesthesia, alert immobility, and passive whole-body rotation (Bland, B.H., Prog. Neurobiol. 26, 1-54, 1986; Shin, J. et al., Proc. Natl. Acad. Sci. U S A. 102, 18165-18170, 2005). In contrast, type I theta rhythms are observed during during locomotion activities, such as walking or running (Bland, B.H., Prog. Neurobiol. 26, 1-54, 1986; Shin, J. & Talnov, A., Brain Res. 897, 217-221, 2001). Also, urethane anesthesia, alert immobility, and passive whole-body rotation were used to record cholinergic type II theta rhythms from wild-type and PLC-β4^{-/-} mice, and medial septum-directed, shRNA-mediated PLC-β4 knockdown mice. Non-cholinergic type I theta rhythms were recorded from mice walking in an open chamber or running on a wheel. The same mice were analyzed in each setting.

### <2-4> Analysis on theta rhythm from PLC-β4 ^{-/-} mice

As a first step to observing non-cholinergic type I theta rhythms when PLC-β4 ^{-/-} mice exercise, hippocampal EEG recordings of mice were recorded. As a result, as illustrated in FIG. 2, the rhythms recorded during exercising of PLC-β4 ^{-/-} mice and wild-type mice were similar to each other. Also, in a 4 to 12 Hz of theta band, EEG amplitudes of PLC-β4 ^{-/-} mice and wild-type mice were not significantly different from each other (P > 0.1, Student's t-test)(FIG. 2B).

Also, to characterize cholinergic type II theta rhythm of PLC-β4 ^{-/-} mice in vivo, hippocampus EEG recordings of mice anesthetized with urethane (1 g/kg, i.p.) were performed. As a result, as illustrated in FIG. 2, in both PLC-β4 ^{-/-} mice and wild-type mice, urethane induced intermittent theta rhythms that were clearly evident in hippocampal fissure recordings collected in undisturbed mice (FIG. 2C). However, power spectral analysis showed that theta power of PLC-β4 ^{-/-} mice was smaller than that of wild-type mice by about 30% (FIG. 2D; P < 0.05, Student's *t* test). In addition, cholinergic type II theta rhythms generated during alert immobility or passive-whole-body rotation were smaller in PLC-β4 ^{-/-} mice than in wild-type mice (FIG. 7).

### <Example 3> Analysis on relationship between PLC-β4 ablation in medial septum, anxiety behaviors, and cholinergic theta rhythm

The inventors of the present invention studied whether PLC-β4 deficiency in medial septum leads to increased anxiety behaviors and decreased cholinergic theta rhythms in PLC-β4 ^{-/-} mice, based on references disclosing that the medial septum regulates theta rhythm (Bland, B.H., Prog. Neurobiol. 26, 1-54, 1986), and that PLC-β4 is substantially expressed in the medial septum (Watanabe, M. et al., Eur. J. Neurosci. 10, 2016-2025, 1998).

### <3-1> shRNA expression and verification of shRNA-mediated knockdown of PLC- β4

PLC-β4-specific shRNA and control shRNA were expressed in the pLKO puromycin-resistance vector (MISSION TRC shRNA Target Set; Sigma-Aldrich, St. Louis, MO, USA). Five different pLKO lentiviral vectors encoding PLC-β4-specific shRNA expression cassettes were tested to knock down PLC-β4 expression in NIH3T3 cells [ATCC(http://www.atcc.org/) CRL-1658]. pLKO-control (SHC002) was used as a non-target shRNA. To assess the efficacy of shRNA, NIH3T3 cells were transfected with shRNA-expressing lentiviral vector constructs, and then the level of PLC-β4 expression was determined by Western blot analysis using rabbit anti-PLC-β4(1:200; Santa Cruz Biotechnology). To increase the levels of PLC- β4 expression in these selection experiments, NIH3T3 cells were transfected with the PLC-β4 expression plasmid, pFLAG-CMV2-PLC-β4. Expression levels were normalized to transfection efficiency, determined by co-transfection with a luciferase plasmid. Two of the PLC-β4-targeting shRNA, shPLC-β4-1 [TRCN0000076919: 5'-GCCTCTTCAA AGTAGATGAA T-3'(SEQID NO: 1)] and shPLC-β4-3[TRCN0000076921: 5'-CCGTCTCCTA ATGACCTCAA A-3'(SEQID NO: 2)] reduced the level of PLC-β4 expression in cells cotransfected with lentivirus expression vectors. shPLC-β4-1 was chosen for subsequent *in vivo* experiments.

### <3-2> Production of lentiviral vectors

HEK293T cells [ATCC(http://www.atcc.org/) CRL-1573] were produced by cotranfection with the following three plasmids: (1) a construct expressing the heterologous envelope protein, VSN-G; (2) *a packaging-defective helper construct expressing the gag-pol gene;* and (3) a transfer vector harboring a PLC-β4-specific shRNA sequence. Cells were transfected using Lipofectamine Plus as described by the manufacturer (Invitrogen). Forty-eight hours after transfection, lentivirus-containing culture supernatants were collected, clarified by passing through a 0.45-mm (Nalgene, USA), and stored immediately at a temperature of -70 °C. Titers were determined using a p24 ELISA(Perkin-Elmer Life Science) or by Western blot analysis using a monoclonal anti-p24 antibody obtained through the AIDS Research and Reference Reagent Program. The titer of our preparations was routinely about 10⁶ to 10⁷ transduction unit (TU)/ml before concentration. Infectious lentivirus particles were concentrated by ultracentrifugation (50,000 × 2h) on a 20% sucrose cushion at a temperature of 4 °C.

### <3-3> Lentivirus-mediated knockdown of PLC-β4 in the medial septum in vivo

High-titer, concentrated lentiviral vectors expressing shPLC-β4-1 or control shRNA were prepared and the lentiviral vectors were injected into the medial septum of 10-week-old wild-type mice by stereotaxic injection into medial septum. Thirteen control shRNA mice and 16 mice injected with shPLC-β4-1 were used in this study. Four weeks after injections, mice were tested using the three anxiety tests, and then hippocampal EEGs were recorded as described below. After behavioral tests and EEG recording, mice were killed and evaluated immunohistochemically to assess the decrease of endogenous PLC-β4 expression in the medial septum.

As a result, in shPLC-β4-1 infected neuronal cells of the medial septum, PLC-β4 staining was substantially reduced, whereas neuronal cells from control shRNA mice showed normal PLC-β4 expression. Accordingly, it was confirmed that lentiviruses expressing shPLC-β4-1 substantially reduce endogenous PLC-β4 expression in medial septal neurons.

### <3-4> Tissue treatment and immunostaining for detection of PLC-β4 knockdown

Tissues were processed and immunostained as previously described (Kim, D.S. et al., J. Comp. Neurol. 511, 581-598, 2008). In detail, the animals were perfused transcardially with phosphate-buffered saline (PBS) by 4% paraformaldehyde in 0.1 M phosphate buffer (PB) pH 7.4). The brains were removed and postfixed in the same fixative for 4 h. Brain tissues were cryoprotected by infiltration with 30% sucrose overnight. Thereafter, the entire medial septal area was frozen and sectioned with a cryostat into 30 um sections and the sections were placed in six-well plates containing PBS. Every sixth section in the series throughout the entire medial septal area from selected animals was used for the immunofluorescence study. The presence and absence of PLC-β4 expression in wild-type and PLC-β4^{-/-} mice, and morphological changes induced by shPLC-β4 in PLC-β4-positive neurons of the medial septum were evaluated by double immunofluorescence staining for mice anti-neuronal nuclei (NeuN) IgG) (1:100; Chemicon, CA, USA) and rabbit anti-PLC β4 IgG(1:100; Chemicon, CA, USA). Brain tissues were incubated in the mixture of antisera overnight at room temperature. After washing three times with PBS (10 min each), sections were incubated in a mixture containing both Cy2-conjugated goat anti mouse IgG (1:200; Amersham, PA, USA) and Cy3-conjugated goat anti-rabbit IgG (1:200; Amersham, PA, USA) for 1 h at room temperature. Sections were mounted in Vectashield mounting media with or without DAPI(Vector, USA). Images were captured and analyzed using an Olympus DP50 digital camera and Viewfinder Life Version 1.0 software, or Olympus FluoView™ FV1000 Confocal Microscope System. Figures were prepared using Adobe Photoshop 7.0(San Jose, CA). Manipulation of images was restricted to threshold and brightness adjustments applied to the entire image.

### <3-5> Analysis on the relationship among PLC-β4 ablation and anxiety behaviors and cholinergic theta rhythm

Lentiviral vectors expressing PLC-β4 targeting shRNA (shPLC-β4) or control shRNA were delivered to the medial septum of 10-week-old wild-type mice.

In postmortem examinations of brains, PLC-β4 staining in neuronal cells of the medial septum of mice infected with shPLC-β4 lentivirus was substantially reduced compared to that in the mice infected with control lentivirus.

Also, it is also investigated whether lentivirus-mediated selective knockdown of PLC-β4 expression in medial septal neurons attenuated cholinergic theta rhythms and/or increased anxiety levels, thus replicating the phenotype of PLC-β4 ^{-/-} mice.

As a result, cholinergic theta rhythms recorded during urethane anesthesia were attenuated in wild-type mice that were infected with lentivirus encoding shPLC-β4 compared with wild-type mice that were infected with control shRNA (FIGS. 3C and 3D), whereas non-cholinergic theta rhythms observed during locomotion remained intact (FIGS. 3A and 3B).

Also, shPLC-β4 mice and control shRNA mice were subjected to the three anxiety behaviors assays. In the open-field assay, shPLC-β4 mice showed no significant difference in the total amount of locomotion activities in the open field compared with control shRNA mice (FIG. 4A; *P* > 0.05, Student's *t*-test). However, shPLC-β4 mice crossed the center of the open field less often than did control shRNA mice (FIG. 4B; *P* > 0.05, Student's *t-*test). Thus, a ratio of the number of entries into the central part of a testing arena to the locomotor activity was enhanced in shPLC-β4 mice than in control shRNA mice (FIG. 4D; *P* < 0.001, Student's *t*-test).

Also, in the elevated plus-maze test shRNA mice made fewer entries into the open arms compared with control shRNA mice (FIG. 4E; *P* < 0.001, Student's *t*-test); the total number of entries did not differ between the two groups (FIG. 4F; *P* = 0.128, Student's *t*-test).

Also, in the light/dark box test, shPLC-β4 mice made fewer transition from the dark to the light compartment compared with control shRNA mice (FIG. 4G; *P* = 0.01, Student's *t*-test). shPLC-β4 mice stayed a significantly short time in the light chamber than did control shRNA mice (FIG. 4H; *P* = 0.047, Student's *t*-test). These results confirm that attenuated cholinergic theta rhythm and increased anxiety behavior phenotypes of PLC-β4 ^{-/-} mice were attributable to the elimination of PLC-β4 proteins from medial septum.

### <Example 4> Rescue of PLC-β4 ^{-/-} cholinergic theta rhythm and anxiety by rivastigmine

The inventors of the present invention investigated whether increased anxiety of PLC-β4 ^{-/-} mice stems from attenuated cholinergic type II theta rhythm based on the result that PLC-β4 ^{-/-} mice show attenuated cholinergic type II theta rhythms, whereas non-cholinergic, serotonine-related type I theta rhythms of PLC-β4 ^{-/-} mice. To this end, whether increasing acetylcholinergic transmission in PLC-β4 ^{-/-} mice could rescue the attenuated amplitude of cholinergic theta rhythms, and thereby normalize anxiety behaviors was investigated. To this end, the cholinergic-enhancing drug, rivastigmine (Van Dam, D. et al., Psychopharmacology 180, 177-190, 2005; Cerbai, F. et al., Eur. J. Pharmacol. 572, 142-150, 2007), an acetylcholinesterase inhibitor known to increase the acetylcholinergic transmission in the hippocampal pathway were each injected into PLC-β4 ^{-/-} mice.

For statistic analysis, differences between groups were compared using Student's *t* test after confirming that data sets were normally distributed. Behavioral data for PLC-β4^{-/-} mice, wild-type mice, and rivastigmine-administered PLC-β4^{-/-} mice were analyzed by ANOVA followed by Tukey's post-hoc test to determine differences among groups.

### <4-1> Administration of rivastigmine

rivastigmine (Novartis Pharma, Basel, Switzerland), which is a cholinergic enhancer, was dissolved in saline. Animals were injected intraperitoneally (i.p.) with 0.5 mg/kg rivastigmine 60 min before the start of anxiety behavioral test (total volume, 5 ml/kg). This dose of rivastigmine was chosen based on the fact that higher dose does not produce an useful effect, which was found based on a previously published microdialysis study that discloses 0.5 mg/kg of rivastigmine increases a hippocampus acetylcholine concentration to about 100% (Kosasa, T., et al., Eur. J. Pharmacol. 380, 101-107, 1999; Scali, C. et al., J. Neural. Transm. 109, 1067-1080, 2002; Liang, Y.Q. & Tang, X.C., Acta. Pharmacol. Sin. 27, 1127-1136, 2006), and a reverse U-shaped dose-response curve of cholinomimetics (Van Dam, D. et al., Psychopharmacology 180, 177-190, 2005). Both PLC-β4 ^{+/+}(WT) and PLC-β4 ^{-/-}(KO) mice were randomly assigned to one of the following treatment groups: (1) WT-sham (wild-type mice with intraperitoneal injection of saline; n = 10), (2) KO-sham (PLC-β4 ^{-/-} mice with intraperitoneal injection of saline; n = 10), and (3) KO-rivastigmine (PLC-β4 ^{-/-} mice with intraperitoneal injection of rivastigmine; n = 10). rivastigmine is a second-generation carbamate-based reversible non-competitive AChE and butyrylChE (BuChE) inhibitor. The reason for choosing the inhibitor is that an inhibitory effect sustains for a long period of time, the inhibitor is specific to the brain (Enz, A. et al., Prog. Brain Res. 98, 431-438, 1993), and the inhibitor selectively increases cholin activity in hippocampus and cortex (Weinstock, M., et al., J. Neural Transm. Suppl. 43, 219-225, 1994).

### <4-2> Restoration of cholinergic theta rhythm by administration of rivastigmine

To experimentally investigate the relationship between cholinergic theta rhythm and anxiety, the following three mice groups were placed in a novel environment and then EEGs from these groups were recorded: (1) PLC-β4 ^{-/-} mice injected with rivastigmine, (2) PLC-β4 ^{-/-} mice injected with saline, and (3) wild-type mice injected with saline. In this regard, a novel environment model, which induces alert-immobility states and evokes anxiety, was chosen as an optimal condition among several conditions (for example, urethane anesthesia, alert immobility, and passive whole-body rotation) known to generate cholinergic theta rhythm in mice cholinergic theta rhythm.

As a result, the amplitude of non-cholinergic type I theta rhythms recorded during locomotion (i.e. walking) in the new open field was not significantly different among the three groups (FIGS. 5A and 5B). However, in saline-injected PLC-β4 ^{-/-} mice, cholinergic theta rhythms were attenuated compared with saline-injected wild-type mice (FIGS. 5C and 5D). Also, the amplitude of the cholinergic theta rhythms of PLC-β4 ^{-/-} mice injected with rivastigmine (0.5 mg/kg, i.p.) was restored to a level similar to that of the wild-type mice injected with saline (FIGS. 5C and 5D). Accordingly, these results suggest that increasing an acetylcholine level in hippocampus restores the attenuated cholinergic theta rhythms in PLC-β4 ^{-/-} mice.

### <4-3> Attenuation of anxiety behaviors by administration of rivastigmine

Whether rivastigmine administration could also rescue the increased anxiety phenotype of PLC-β4 ^{-/-} mice was investigated. To this end, rivastigmine (0.5 mg/kg, i.p.) was injected into PLC-β4 ^{-/-} mice and, 60 min after injection, the rivastigmine treated group was subjected to three anxiety behavioral assays. In each of the three anxiety tests, the rivastigmine treated group showed a reversal of the increased anxiety phenotype of the PLC-β4 ^{-/-} mice (FIG. 6).

In the open-field test, PLC-β4 ^{-/-} mice injected with saline showed an overall decrease in locomotion (FIG. 6A; ANOVA, *F*₂,₄₅ = 6.96, *P* = 0.0023) and moved less in the center of the open field than did saline injected wild-type littermates (FIG. 6B; ANOVA, *F*₂,₄₅ = 4.34, *P* = 0.019). However, the rivastigmine injected PLC-β4 ^{-/-} mice showed wild-type levels of locomotion in the center of the open field as well as wild-type levels of total locomotion activities (FIGS. 6A and 6B). Also, Rivastigmine administration also restored the amount of time spent in the central sector of the open field to levels comparable with those in wild-type mice injected with saline (FIG. 6C; ANOVA, *F*₂,₄₅ = 5.46, *P* < 0.01). Also, the rivastigmine administration reduced thigmotaxis index to the same levels as those in wild-type mice injected with saline (FIG. 6D; ANOVA, *F*₂,₄₅ = 7.46, *P* < 0.001).

In the elevated plus-maze, the saline injected PLC-β4 ^{-/-}mice made fewer entries into the open arms, whereas the behavior of the rivastigmine-treated group was indistinguishable from wild-type mice injected with saline (FIG. 6E; ANOVA, *F*₂,₄₅ = 4.2, *P* = 0.021). Total entries into the closed and open arms did not differ among the three groups (FIG. 6F; ANOVA, *F*_{2,45} = 0.43, *P* = 0.44).

In the light/dark box test, PLC-β4 ^{-/-} mice injected with saline made fewer transitions between light and dark boxes (FIG. 6G; ANOVA, *F*₂,₂₁ = 4.5, *P* = 0.024) and spent significantly less time in the light chamber (FIG. 6H; ANOVA, *F*₂,₂₁ = 4.8, *P* = 0.019). In both cases, the behavior of rivastigmine-treated PLC-β4 ^{-/-} mice was indistinguishable fro that of wild-type mice injected with saline.

Accordingly, it can be confirmed that rivastigmine treatment is sufficient to restore normal cholinergic theta rhythms and normal anxiety behaviors in PLC-β4 ^{-/-} mice, and cholinergic theta rhythms play a critical role in suppressing anxiety.

### INDUSTRIAL APPLICABILITY

The present invention may be used in screening an anxiety behavioral subject that is suitable for administration of a cholinergic drug, and also in monitoring prognosis after administration of a cholinergic drug to the anxiety behavioral subject, by using cholinergic type II theta rhythms.

## Claims

1. A method of evaluating suitability for drug therapy for the prevention or treatment of an anxiety disorder in a non-human subject, the method comprising:
1) recording a cholinergic type II theta rhythm from a non-human subject that has an anxiety disorder;
2) screening a subject that has an attenuated amplitude of the cholinergic type II theta rhythm compared with that of a normal subject; and
3) determining the subject as a subject that requires a treatment with a cholinergic enhancer as a therapeutic agent for the anxiety disorder.

2. The method of claim 1, wherein the cholinergic type II theta rhythm of step 1) is recorded by electroencephalogram (EEG).

3. The method of claim 1, wherein the cholinergic enhancer of step 3) is one selected from the group consisting of an allosteric sensitization agent, an cholinergic receptor activation agent, an agent for activating intracellular pathways between related cells through second messenger cascades, and an acetylcholinesterase inhibitor.

4. The method of claim 1, wherein the cholinergic enhancer of step 3) is selected from the group consisting of rivastigmine, donepezil, galantamine, tacrine, metrifonate, physostigmine, neostigmine, pyridostigmine, ambenonium, demarcarium, edrophonium, huperzine A, and onchidal.

5. A kit for evaluating suitability for use of a cholinergic enhancer as a drug for the prevention or treatment of anxiety disorders, the kit comprising an EEG recorder for analyzing cholinergic type II theta rhythms.

6. A method of monitoring prognosis of an anxiety disorder after the administration of a cholinergic enhancer to a non-human subject, the method comprising:
1) administrating an effective amount of a cholinergic enhancer to a non-human subject having an anxiety disorder;
2) recording a cholinergic type II theta rhythm from the non-human subject; and
3) evaluating a restoration level of the amplitude of the cholinergic type II theta rhythm compared with that of a normal subject as a recovery level of the anxiety disorder.

7. A kit for monitoring prognosis of an anxiety disorder after the administration of a cholinergic enhancer, the kit comprising an EEG recorder for analyzing cholinergic type II theta rhythms.

8. A method of diagnosing an anxiety disorder in a non-human subject, the method comprising:
1) recording a cholinergic type II theta rhythm from the non-human subject; and
2) determining a subject that has a lower amplitude of a cholinergic type II theta rhythm than that of a normal subject as a subject that is prone to develop an anxiety disorder.

9. A method of screening a drug for the prevention or treatment of anxiety disorders in a non-human subject by using the cholinergic type II theta rhythms:
1) administering a test material to the non-human subject having an anxiety disorder;
2) measuring a cholinergic type II theta rhythm of the non-human subject; and
3) selecting a material that attenuates the anxiety disorder by comparing an amplitude of the cholinergic type II theta rhythm of the non-human subject with that of a normal subject.
